# EUROPÄISCHE PATENTSCHRIFT

(11) **EP 2 922 484 B1**
(45) Veröffentlichungstag und Bekanntmachung des Hinweises auf die Patenterteilung: **25.07.2018**
(21) Anmeldenummer: 14700660.5
(22) Anmeldetag: 15.01.2014
(51) Int. Cl.: A61B 17/86, A61B 17/80

(54) **CHIRURGISCHE KNOCHENSCHRAUBE SOWIE IMPLANTATIONSSYSTEM**
SURGICAL BONE SCREW AND IMPLANT SYSTEM
VIS CHIRURGICALE D'OSTÉOSYNTHÈSE ET SYSTÈME D'IMPLANTATION

(30) Priorität: 15.01.2013 DE 102013100362
(43) Veröffentlichungstag der Anmeldung: 30.09.2015
(60) Teilanmeldung: 18161579.0
(73) Patentinhaber: Zimmer GmbH, 8404 Winterthur (CH)
(72) Erfinder: PECH, Uwe, 78532 Tuttlingen (DE); KOTULJAC, Vladko, 72355 Schömberg (DE)
(74) Vertreter: Manitz Finsterwald Patentanwälte PartmbB
(86) Internationale Anmeldenummer: PCT/EP2014/050725
(87) Internationale Veröffentlichungsnummer: WO 2014/111432

(56) Entgegenhaltungen:
- EP-A1- 1 493 399
- EP-A2- 1 273 273
- WO-A1-2004/086990
- DE-C1- 3 241 963

## Beschreibung

Die Erfindung betrifft ein Implantationssystem gemäß Anspruch 1.

Heute werden in der Chirurgie sogenannte winkelstabile Fixationssysteme eingesetzt, umfassend eine Knochenplatte mit mindestens einem Innengewindeloch sowie eine Knochenschraube, die in verschiedenen Winkeln in das Innengewindeloch einschraubbar und mit der Knochenplatte verblockbar ist.

Ein derartiges Funktionsprinzip ist beispielsweise aus der EP 1 143 867 B1 bekannt. Das bekannte Fixationssystem beruht auf der Idee, beim Einschrauben der Knochenplatte durch Materialumformung eine Gewindeverbindung zwischen Knochenschraube und Knochenplatte zu erzeugen. Nachteilig bei dem bekannten System ist, dass die Verbindung aus Knochenschraube und Knochenplatte zur Zentrierung neigt, so dass ein beabsichtigter Einbringwinkel in der Praxis sich nur schwer einstellen bzw. erreichen lässt.

Außerdem wird auf DE 43 43 117 C2 verwiesen.

Ein alternatives Fixationssystem ist aus der WO 2004/086990 A1 bekannt. Bei der bekannten Knochenschraube sind am Umfang des nicht rotationssymmetrischen Schraubenkopfes in Umfangsrichtung beabstandete Abflachungen vorgesehen, wobei die Verblockung aus einer Keilverklemmung mit der Knochenplatte resultiert. Auch hier scheint die Verblockungswirkung verbesserungswürdig.

Zum weiteren Stand der Technik werden die US 2005/0070904 A1, die FR 2910800 A1, die US 2007/162018 A1, US 2006/009771 A1, die US 2008/140130 A1, die DE 20 2005 014850 U1, die EP 1 493 399 A1, die EP 1 273 273 A2 sowie die DE 32 41 963 C1 genannt.

Der Erfindung liegt die Aufgabe zugrunde, ein winkelstabiles Fixationssystem (Implantationssystem) mit einer verbesserte Knochenschraube anzugeben, die auch unter großen Einschraubwinkeln, von vorzugsweise größer als 15°, insbesondere von 20°, einbringbar und in dieser Winkelposition sicher fixierbar ist. Insbesondere soll sich die Knochenschraube dabei durch eine vergleichsweise leichte Einbringbarkeit und vor allem dadurch auszeichnen, dass sie schnell greift, d.h. in blockierte Wechselwirkung mit der Knochenplatte tritt. Insbesondere sollen Selbstzentrierungseffekte beim Zusammenwirken mit einer Knochenplatte vermieden werden.

Diese Aufgabe wird durch ein Implantationssystem (Fixationssystem) mit den Merkmalen des Anspruchs 1 gelöst. Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Die Erfindung hat erkannt, dass es zur Lösung vorstehender Aufgabe notwendig ist, eine konkave Hüllkontur des Schraubenkopfes mit einer unterbrochenen Rillenstruktur zu kombinieren, um den gewünschten Effekt eines sicheren Haltes der Knochenschraube in einer Knochenplatte in unterschiedlichen Winkeln oder unmittelbar im Knochen, insbesondere in der Kortikalis, zu gewährleisten. Die konkave Hüllkontur bewirkt dabei in Kombination mit der mindestens einen aus der Unterbrechung der Rillenstruktur, genauer des Flankenstrukturabschnittes, resultierenden Erhebung, insbesondere mit der Vielzahl von Erhebungen, einen schnellen und einen innigen Kontakt zwischen den Erhebungen und der Knochenplatte und lässt darüber hinaus größere Einbringwinkel zu als im Stand der Technik - mit anderen Worten kann durch die Kombination aus der mindestens einen Erhebung und der konkav rotationssymmetrischen Hüllkontur des Schraubenkopfes auch bei vergleichsweise großen Schrägstellungen bzw. großen Einbringwinkeln der Knochenschraube in den Schrau benkopf ein stabiler Halt gewährleistet werden.

Dabei ist unter dem Begriff "Rillenstruktur" insbesondere eine solche Struktur zu verstehen, die eine oder mehrere Rillen umfasst, die zumindest im Wesentlichen in Umfangsrichtung verlaufen.

Wenn die Knochenschraube mit einer Knochenplatte verwendet wird, dann trifft bei einer konkaven Hüllkontur der Schraubenkopf, d.h. der Flankenstrukturabschnitt der Rillenstruktur, erst bei einem größeren Einbringwinkel auf die Kante des in der Durchgangsöffnung der Knochenplatte ausgebildeten Gewindes als bei einer zylindrischen oder konischen Hüllkontur. Mit anderen Worten kann die erfindungsgemäße Knochenschraube unter größeren Winkeln eingedreht werden als Knochenschrauben mit zylindrischer oder konischer Hüllkontur, bevor wegen des unerwünschten Auftreffens der Struktur des Schraubenkopfes auf die Kante der Durchgangsöffnung die Gefahr einer Selbstzentrierung oder eines unkontrollierten Wegdrückens der Platte droht oder ein Einschrauben in die Platte überhaupt nicht mehr möglich ist. Hierauf wird an anderer Stelle noch näher eingegangen.

Dabei wird unter dem Flankenstrukturabschnitt eine langgestreckte (erfindungsgemäß unterbrochene) erhabene Kontur verstanden, die zwei seitliche, insbesondere aufeinander zu geneigte (im Wesentlichen in axialen Richtungen weisenden) Flanken aufweist - also ein (unterbrochener) Wulst, der sich mit oder ohne Steigung und um die Knochenschraube erstreckt, insbesondere um mehr als eine Umdrehung. Der Flankenstrukturabschnitt wird nachstehend auch einfach als Flankenabschnitt bezeichnet.

Im Hinblick auf die konkrete Ausbildung der rotationssymmetrischen Hüllkontur mit ihrem konkaven Verlauf gibt es unterschiedliche Möglichkeiten. So ist es möglich, einen mathematisch stetigen konkaven Verlauf zu erzielen. Die resultierende Hüllkontur kann in diesem Fall von einer gekrümmten mathematischen Funktion beschrieben werden, die um die Längsmittelachse der Knochenschraube (mit Radialabstand zu dieser) rotiert ist. Bei einer alternativen Ausführungsform kann die Hüllkontur einen nicht stetigen, d.h. gestuften bzw. geknickten Verlauf haben, also einen nicht stetigen Übergang zwischen zwei konkaven bzw. kegelstumpfförmigen Abschnitten, wobei sich der Konus- bzw. Kegelwinkel der beiden axial aneinander angrenzenden Abschnitte unterscheidet.

Wie später noch erläutert werden wird, resultiert die Unterbrechung der Rillenstruktur, genauer gesagt des erhabenen Flankenstrukturabschnittes (Wulstabschnittes) der Rillenkontur, welcher auf zumindest einer Flankenseite, insbesondere auf zwei Flankenseiten eine Vertiefung (Rille) begrenzt, aus dem Vorsehen einer Art winklig zur Umfangserstreckung verlaufenden Einkerbung oder Nut, die in einer Projektion auf eine die Längsmittelachse der Knochenschraube enthaltende Ebene sich zumindest näherungsweise in axialer Richtung erstreckt. Insbesondere sind mehrere solcher Einkerbungen in Umfangsrichtung beabstandet zueinander angeordnet, so dass eine Vielzahl von in Umfangsrichtung beabstandeten Erhebungen resultieren. Durch das Vorsehen einer Mehrzahl von Erhebungen lässt sich die Schraube besonders leicht eindrehen und greift besonders schnell. Ein Ausbrechen der Schraube wird vermieden.

Wenn im Folgenden in Verbindung mit der Längserstreckung der Einkerbungen von einer axialen Richtung oder Axialrichtung die Rede ist, dann ist hierunter eine axiale Richtung in der vorstehend erwähnten Projektion zu verstehen.

Insbesondere ist vorgesehen, dass sich Erhebungen nicht nur in einer Ebene befinden, sondern wenn zusätzlich zu in Umfangsrichtung beabstandeten Erhebungen auch in axialer Richtung beabstandete Erhebungen vorgesehen sind.

Wie später noch erläutert werden wird, ist insbesondere vorgesehen, dass die Erhebungen eine Pyramiden- oder Walmdachkontur bzw. -geometrie aufweisen. Hierunter wird eine Form verstanden, bei der die Erhebungen vier winklig zueinander angeordnete und in Richtung zu einer Erhebungsmitte geneigte Flanken aufweisen. Mehrere in axialer Richtung beabstandete Erhebungen können je nach Ausgestaltung der Rillenstruktur bzw. des Flankenabschnittes daraus resultieren, dass ein über mehr als eine Windung gewendelter Flankenabschnitt vorgesehen ist oder dass zwei zueinander parallele ringförmige Flankenabschnitte vorgesehen sind, die sich in zueinander parallelen Radialebenen befinden oder winklig hierzu angeordnet sein können, wie später noch erläutert wird.

Die Erhebungen können auch eine Pyramidenstumpfform, eine Keilform oder eine Keilstumpfform besitzen.

Neben der Anwendung der erfindungsgemäßen chirurgischen Knochenschraube in einem winkelstabilen Implantationssystem mit einer Knochenplatte eignet sich die erfindungsgemäße Knochenschraube durch die spezielle Ausgestaltung des Knochenschraubenkopfes auch zur unmittelbaren Verankerung im Knochen, wobei sich der Schraubenkopf durch seine mindestens eine, vorzugsweise durch die mehreren Erhebungen durch die Kortikalis fräst und sich selbsttätig im Knochen verankert. Durch ein mögliches Verwachsen des Knochens in einen Bereich zwischen den Erhebungen wird die Stabilität der Verbindung weiter erhöht. Im vorgenannten Fall kann die Knochenschraube als sogenannte intramedulläre Verriegelungsschraube eingesetzt werden. Auch ist es denkbar, die Knochenschraube als Kompressionsschraube vorzusehen, wobei die Knochenschraube in diesem Fall voneinander unterschiedliche Steigungen aufweisende Gewinde aufweisen sollte. Insbesondere kann in diesem Fall vorgesehen sein, dass die Rillenstruktur des Schraubenkopfes , insbesondere mehrere durch das Unterbrechen des Flankenstrukturabschnitts gebildete Erhebungen, ein Außengewinde bilden, dessen Steigung sich von der Steigung eines Gewindes des Schraubenschaftes unterscheidet.

Die nach dem Konzept der Erfindung ausgebildete Schraube kann aus Titan, Stahl oder Kunststoff, insbesondere PEEK ausgebildet werden. Im Falle des Vorsehens einer zur Knochenplatte gehörigen Schraube kann die Knochenplatte entweder aus dem gleichen Material wie die Schraube, insbesondere jedoch aus einem unterschiedlichen, weicheren Material ausgebildet sein.

Wie eingangs erwähnt, gibt es im Hinblick auf die Ausgestaltung der Rillenstruktur unterschiedliche Möglichkeiten. So kann diese in der Art eines Außengewindes ausgebildet sein, also derart, dass der Flankenabschnitt gewendelt ist, und zwar im einfachsten Fall einmal um den Schraubenkopf herum, wobei insbesondere bei einerseits schwierigerer Herstellung, andererseits aber zur Erhöhung der Anzahl der tragenden Elemente vorgesehen ist, dass sich der gewendelte Flankenabschnitt über mehr als den einfachen Schraubenkopfumfang, insbesondere über mindestens den 1,5-fachen, insbesondere über mindestens den doppelten oder dreifachen Umfang des Schraubenkopfes erstreckt. Insbesondere ist vorgesehen, dass der Flankenabschnitt einen Umfangswinkel mit Axialabstand mehrfach passiert. Alternativ zu einem gewendelten Flankenabschnitt kann dieser auch steigungsfrei ausgebildet sein, also einen durch mindestens eine Unterbrechung unterbrochenen Ring bilden, wobei insbesondere vorgesehen ist, dass mehrere solcher Ringe vorgesehen sind, die insbesondere parallel zueinander angeordnet sind. Dabei ist es gemäß einer ersten Alternative möglich, dass sich die ringförmigen (unterbrochenen) Flankenstrukturabschnitte in zueinander parallelen Radialebenen befinden. In diesem Fall sind die Flankenstrukturabschnitte kreisringförmig. Bei einer alternativen Ausführungsform sind die Flankenstrukturabschnitte oval und damit winklig zu vorgenannten Radialebenen angeordnet.

Insbesondere ist eine Ausführungsform der Knochenschraube vorgesehen, bei der die Hüllkontur des Schraubenkopfes, insbesondere axial durchgängig, zumindest jedoch über einen Axialabschnitt, insbesondere über den größten Teil der Axialerstreckung des Schraubenkopfes, konkav ausgebildet ist.

Insbesondere ist vorgesehen, dass bei einem stetigen Hüllkonturmantelflächenverlauf die konkave Ausformung in einer Längsschnittansicht einen Teilkreisbogen beschreibt oder umfasst, wobei der Radius des Teilkreisbogens insbesondere aus einem Wertebereich zwischen 0,5mm und 7mm, insbesondere zwischen 1mm und 6mm, insbesondere zwischen 2mm und 4mm gewählt ist und insbesondere zumindest näherungsweise 3mm beträgt.

Die Hüllkontur kann mindestens zwei Kegelstumpfabschnitte umfassen oder von mindestens zwei Kegelstumpfabschnitten gebildet sein, wobei insbesondere die Kegelstumpfabschnitte voneinander unterschiedliche Kegelwinkel aufweisen.

Bei der eingangs beschriebenen alternativen Ausführungsform mit nicht stetiger Hüllkontur ist insbesondere vorgesehen, dass die kegelstumpfförmigen Mantelflächenabschnitte in einer Längsschnittansicht Tangenten an einen Teilkreisbogen bilden, insbesondere mit einem Radius aus den zuvor genannten Wertebereichen oder mit dem zuvor genannten Wert.

Insbesondere ist vorgesehen, dass nicht nur in Umfangsrichtung beabstandete Erhebungen vorgesehen sind, sondern dass, insbesondere an zumindest einem Umfangswinkel, mehrere, insbesondere zwei oder mehr als zwei, axial beabstandete Erhebungen vorgesehen sind, was einerseits dadurch erreicht werden kann, dass sich ein gewendelter Flankenstrukturabschnitt mehr als einmal in Umfangsrichtung um den Schraubenkopf erstreckt, oder andererseits dadurch, dass mindestens zwei parallel zueinander angeordnete ringförmige Flankenstrukturabschnitte vorgesehen sind, die jeweils mindestens einmal, vorzugsweise mehrfach, unterteilt sind.

Insbesondere ist vorgesehen, dass die Erhebungen in Umfangsrichtung durch insbesondere als Einfräsungen ausgeführte Einkerbungen unterbrochen sind, die sich insbesondere bis zum axial hinteren Ende des Schraubenkopfes erstrecken.

Unter dem axial hinteren Ende des Schraubenkopfes ist das freie Ende des Schraubenkopfes zu verstehen.

Wie später noch erläutert wird, ist insbesondere vorgesehen, dass die Einkerbungen in Umfangsrichtung geneigte Flanken aufweisen, um so insbesondere eine Pyramiden- oder Walmdachform der Erhebungen zu erzielen. Gemäß einer ersten Alternative können sich die Einkerbungen entlang der Längserstreckung der Knochenschraube, d.h. axial erstrecken, oder in Umfangsrichtung geneigt ausgeführt sein. Insbesondere ist vorgesehen, dass eine Vielzahl solcher Einkerbungen vorgesehen sind, insbesondere mehr als sechs, insbesondere zwölf oder mehr als zwölf. Dabei ist insbesondere vorgesehen, dass (auch) die sich in Umfangsrichtung erstreckenden und insbesondere jeweils eine Rille begrenzenden Flanken des Flankenstrukturabschnittes aufeinander zu geneigt sind. Insbesondere ist vorgesehen, dass, wie erläutert, die Erhebungen neben den zwei aufeinander zu (oberen und unteren) geneigten und sich in der Umfangserstreckung erstreckenden Flanken zwei weitere geneigte Flanken aufweisen, wobei diese Flanken mit ihrer Flächenseite in Umfangsrichtung weisen und jeweils eine Einkerbung begrenzen. Diese beiden Flanken können sich gerade in radialer Richtung erstrecken oder insbesondere hin zu einer Erhebungsmitte geneigt sein, so dass je nach Umfangserstreckung der Erhebung eine Pyramiden- oder Walmdachform der Erhebungen resultiert, insbesondere wenn die Einkerbungen axial verlaufen, d.h. nicht in Umfangsrichtung geneigt sind. Bei in Umfangsrichtung geneigten Einkerbungen resultiert eine entsprechend ihrem Grundriss verschobene Pyramiden- bzw. Walmdachform, wobei der Grundriss der Erhebung dann im Wesentlichen parallelogrammförmig ist, wenn vernachlässigt wird, dass der Umfang der Hüllkontur aufgrund der konkaven Ausgestaltung hin zum hinteren Ende des Schraubenkopfes zunimmt.

Insbesondere ist vorgesehen, dass die jeweils eine Einkerbung begrenzenden Flanken des mindestens einen Flankenstrukturabschnittes unter einem Winkel aus einem Winkelbereich zwischen 40° und 120°, insbesondere von etwa 60°, zueinander geneigt sind.

Insbesondere eine Pyramiden- oder Walmdachform verbessert das schnelle Eingreifen beim Eindrehen der Schraube und erleichtert dieses. Zudem wird ein Ausbrechen der Schraube optimal vermieden und es resultiert eine optimale Kraftverteilung. Durch das Vorsehen einer Pyramiden- oder Walmdachform werden Hebelkräfte beim Eindrehen der Schraube, die üblicherweise zu einer Schraubenzentrierung führen würden optimal vermieden. Ferner wird durch eine Pyramiden- oder Walmdachform ein Explantieren der Schraube erleichtert, da sich die einzelnen Pyramiden bzw. Walmdächer einen eigenen Weg durch die Verankerung fräsen.

Zur Vermeidung von Rupturen bzw. Reizungen von Weichteilen und Sehnen hat es sich als vorteilhaft herausgestellt, eine hinterste Kante des Schraubenkopfes gerundet auszubilden.

Die Durchgangsöffnung kann entweder mit einem Innengewinde, insbesondere einem metrischen Gewinde wie z.B. einem M3-Gewinde, oder mindestens einem, vorzugsweise ausschließlich einem nach radial innen vorstehenden Flankenring versehen sein, um mit den Erhebungen der Knochenschraube in blockierende Wechselwirkungen zu treten.

Vorzugsweise ist das Material der Knochenplatte weicher als das Material für die Knochenschraube. Folglich kommt es beim Eindrehen der Knochenschraube in die Durchgangsöffnung zu einer Verformung oder Umformung des Innengewindes oder des zumindest einen Flankenringes der Durchgangsöffnung durch den Flankenstrukturabschnitt bzw. die Erhebung oder Erhebungen am Kopf der Knochenschraube. Insbesondere können die zusammenwirkenden Strukturen von Schraubenkopf und Durchgangsöffnung hinsichtlich Formgebung und Materialwahl so ausgebildet sind, dass es zu einem Kaltverschweißen oder zu einer einem Kaltverschweißen ähnlichen Verbindung zwischen Schraubenkopf und Durchgangsöffnung kommt, wenn die Schraube in die Durchgangsöffnung eingedreht wird.

Insbesondere sind die zusammenwirkenden Strukturen von Schraubenkopf und Durchgangsöffnung hinsichtlich Formgebung und Materialwahl so ausgebildet, dass trotz der guten Verbindung zwischen Schraubenkopf und Durchgangsöffnung diese Verbindung wieder gelöst und die Schraube ein oder mehrere weitere Male in die Durchgangsöffnung eingedreht werden kann.

Bei dem erfindungsgemäßen System handelt es sich um ein so genanntes polyaxiales Implantations- oder Fixationssystem, das es ermöglicht, die Knochenschrauben jeweils unter einem beliebigem Winkel in die Durchgangsöffnungen der Knochenplatte einzudrehen und für jeden Eindrehwinkel eine winkelstabile Anordnung der Knochenschraube relativ zur Knochenplatte zu realisieren.

Die Durchgangsöffnung kann ausschließlich einseitig oder beidseitig mit einer Senkung versehen werden.

Um eine besonders hohe Stabilität der Verbindung zu erreichen, hat sich als vorteilhaft herausgestellt, die Durchgangsöffnung mit mindestens einer sich in Richtung der Längserstreckung der Durchgangsöffnung erstreckenden Einkerbung zu versehen. Insbesondere ist die Durchgangsbohrung sternförmig ausgefräst. Die Einkerbungen können die gleiche Tiefenerstreckung haben oder eine voneinander unterschiedliche. Auch ist es denkbar, dass die Tiefenerstreckung, d.h. die Radialerstreckung der Einkerbungen konstant ist oder im Falle eines schrägen Einkerbungsverlaufs sich über die Axialerstreckung verändert.

Vorzugsweise ist vorgesehen, dass die Durchgangsöffnung und/oder die zumindest eine Einkerbung zylindrisch ist, d.h. sich nicht verjüngt.

Des Weiteren ist vorzugsweise vorgesehen, dass die mindestens eine Längseinkerbung in einer Ebene, die senkrecht zu einer Längsachse der Durchgangsöffnung verläuft, einen teilkreisförmigen Querschnitt aufweist. Insbesondere ist vorgesehen, dass die Durchgangsöffnung der Knochenplatte mindestens zwei axial benachbarte Flankenringe aufweist, deren Innendurchmesser sich voneinander unterscheidet.

Weitere Vorteile, Merkmale und Einzelheiten der Erfindung ergeben sich aus der Beschreibung möglicher Ausführungsbeispiele sowie anhand der Zeichnungen. Diese zeigen in:
- Fig. 1: einen oberen Abschnitt einer Knochenschraube mit einer konkaven Hüllkontur und mit einem gewendelten Flankenstruktur-abschnitt, der beidseits eine gewendelte Gewinderille begrenzt,
- Fig. 2: eine alternative Ausführungsform einer Schraube, bei der mehrere parallel zueinander angeordnete, kreisringförmige Flankenstrukturabschnitte vorgesehen sind,
- Fig. 3: eine weitere alternative Ausführungsform eines Schraubenkopfes, bei der Einkerbungen in den Flankenstrukturabschnitten durch in Umfangsrichtung geneigte Einkerbungen gebildet sind,
- Fig. 4 - 9: alternative Ausführungsformen von Knochen und Platten zur Wechselwirkung mit den in den Fig. 1 bis 3 gezeigten Knochenschrauben, wobei sich die Knochenplatten durch voneinander unterschiedliche Durchgangsöffnungsgeometrien auszeichnen,
- Fig. 10: eine Draufsicht auf eine Knochenplatte mit Durchgangsöffnung, die sternförmig mit Einkerbungen versehen ist,
- Fig. 11: ein weiteres Ausführungsbeispiel einer erfindungsgemäßen Knochenschraube,
- Fig. 12: ein weiteres Ausführungsbeispiel einer erfindungsgemäßen Knochenschraube,
- Fig. 13: ein weiteres Ausführungsbeispiel einer erfindungsgemäßen Knochenplatte, und
- Fig. 14: eine Darstellung zur Erläuterung der Wirkung einer konkaven Hüllkontur.

In den Figuren sind gleiche Elemente und Elemente mit der gleichen Funktion mit den gleichen Bezugszeichen gekennzeichnet.

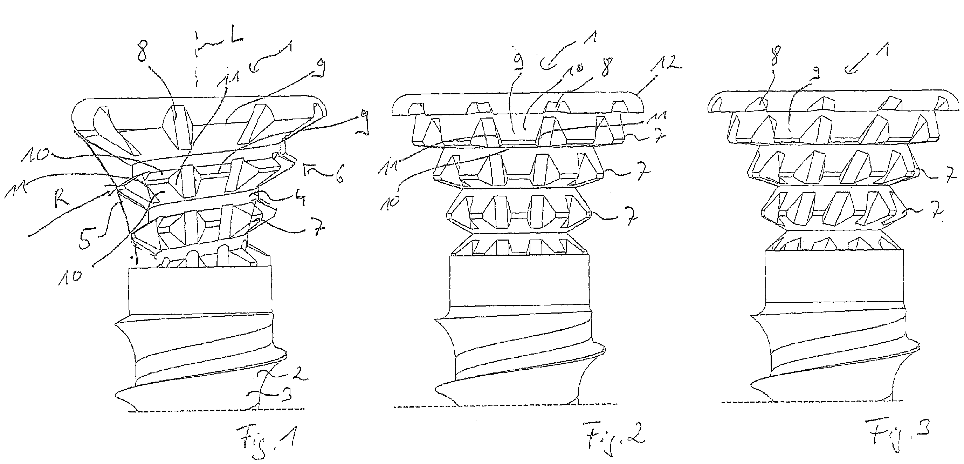

Fig. 1 zeigt eine chirurgische Knochenschraube 1 mit einem ein Knochengewinde 2 tragenden und nur in einem oberen Axialabschnitt dargestellten Schaft 3. Die Knochenschraube 1 weist zudem im Bereich ihres hinteren, in der Zeichnungsebene oberen Endbereichs einen Schraubenkopf 4 auf, der stirnseitig mit einem in den Zeichnungen nicht gezeigten Antrieb, beispielsweise einem Torxantrieb, versehen ist. Der Schraubenkopf 4 zeichnet sich durch eine rotationssymmetrische, konkave Hüllkontur 5 auf, die andeutungsweise in der linken Zeichnungshälfte gezeigt ist. Bei dem Ausführungsbeispiel gemäß Fig. 1 weist die Hüllkontur in axialer Richtung einen stetigen Verlauf auf, kann also von einer um eine Längsmittelachse L der Knochenschraube 1 rotierte, gekrümmte mathematische Funktion beschrieben werden. In dem konkreten Ausführungsbeispiel handelt es sich um einen Teilkreisbogen, hier mit einem Radius R = 3mm. Wesentlich ist die konkave Form, die nicht zwingend einen stetigen Verlauf haben muss, sondern auch von zwei axial aneinander angrenzenden Konus- oder Kegelabschnitten mit einem voneinander unterschiedlichen Konuswinkel gebildet sein oder zwei derartige Konus- oder Kegelabschnitte umfassen kann.

Zu erkennen ist, dass der Schraubenkopf mit einer Rillenstruktur 6 versehen ist, die in dem gezeigten Ausführungsbeispiel an ein Außengewinde erinnert.

Die Rillenstruktur 6 weist dabei einen gewendelten (erhabenen), d.h. eine Steigung aufweisenden Flankenstrukturabschnitt 7 auf, der sich mehrfach um den Umfang des Schraubenkopfes erstreckt. Zu erkennen ist, dass der Flankenstrukturabschnitt 7, hier in regelmäßigen Abständen, unterbrochen ist, und zwar durch sich in axialer Richtung entlang der Knochenschraube 1 erstreckende Einkerbungen 8, wobei jede Einkerbung 8 dem gewendelten Flankenstrukturabschnitt 7 mehrfach (auf unterschiedlichen axialen Höhen) durchsetzt, so dass insgesamt sowohl eine Vielzahl von in Umfangsrichtung beabstandeten als auch eine Vielzahl von in axialer Richtung beabstandeten Erhebungen 9 resultiert, die in dem gezeigten Ausführungsbeispiel, was im Folgenden noch erläutert werden wird, eine Walmdachform besitzen. Die Erhebungen 9 bilden ein Außengewinde bzw. liegen auf einer Linie, wie sie ein Außengewinde definieren würde.

Die Walmdachform resultiert daraus, dass die beiden sich in Umfangsrichtung erstreckenden Flanken 10, die jeweils eine Gewinderille bzw. einen Gewindegang begrenzen, aufeinander zu geneigt sind. Im Wesentlichen rechtwinklig zu diesen beiden Flanken 10 verlaufen zwei jeweils eine Einkerbung 8 begrenzende weitere Flanken 11, wobei die beiden weiteren Flanken 11 einer Erhebung ebenfalls aufeinander zu geneigt sind. Insgesamt sind alle vier Flanken 10, 10, 11, 11 der Erhebungen hin zu einer Erhebungsmitte, d.h. aufeinander zu, geneigt. Für den Fall eines größeren Neigungswinkels der beiden Flanken 11 und/oder für den Fall einer kürzeren Umfangserstreckung der Erhebungen 9 aufgrund des Vorsehens von mehr Einkerbungen 8 kann die gezeigte, insbesondere vorgesehene Walmdachform auch in eine Pyramidenform münden, die im Vergleich zur Walmdachform eher spitz hin zu einem Punkt zuläuft.

Das in Fig. 2 gezeigte Ausführungsbeispiel einer Knochenschraube 1 unterscheidet sich von dem in Fig. 1 dargestellten zuvor beschriebenen Ausführungsbeispiel im Wesentlichen nur dadurch, dass nicht nur ein einziger, gewendelter Flankenstrukturabschnitt 7 vorgesehen ist, sondern mehrere axial benachbarte und sich jeweils in einer Radialebene befindende Flankenstrukturabschnitte 7, also Flankenstrukturabschnitte ohne Gewindesteigung. Auch bei diesem Ausführungsbeispiel erstrecken sich die Einkerbungen 8 bis zum axial hinteren Ende der Knochenschraube 1 und verlaufen in axialer Richtung, so dass die walmdachförmigen Erhebungen 9 resultieren, die in axialer Richtung von den Flanken 10 und in Umfangsrichtung von den Flanken 11 begrenzt sind.

Wie auch bei dem Ausführungsbeispiel gemäß Fig. 1 ist eine hinterste Kante 12 der Knochenschraube 1 zur Vermeidung von Gewebeverletzungen gerundet ausgeführt.

Das Ausführungsbeispiel der in Fig. 3 gezeigten Knochenschraube 1 unterscheidet sich wiederum von dem in Fig. 2 dargestellten Ausführungsbeispiel lediglich dadurch, dass die Einkerbungen 8 nicht gerade in axialer Richtung verlaufen, sondern in Umfangsrichtung geneigt sind, wodurch die Grundrisse der walmdachförmigen Erhebungen 9 in der Art eines Parallelogramms verschoben sind. Das Ausführungsbeispiel gemäß Fig. 3 kann alternativ zu der Mehrzahl von Flankenstrukturabschnitten 7 auch mit einem einzigen, gewendelten Flankenstrukturabschnitt 7, wie in Fig. 1 gezeigt, ausgebildet werden.

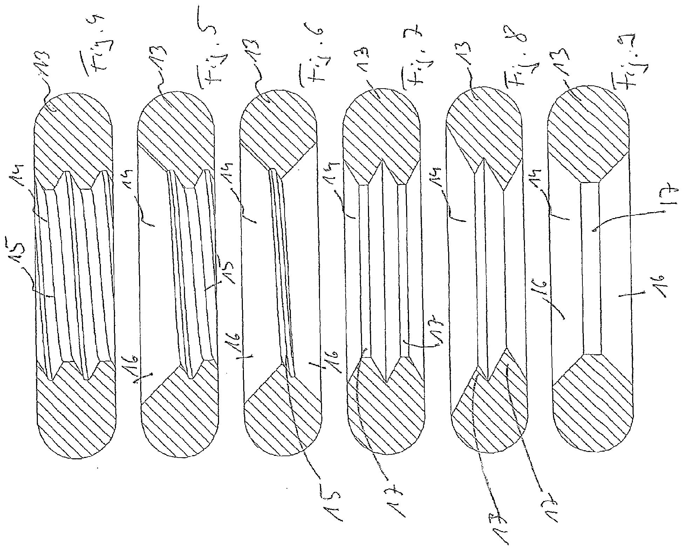

In den Fig. 4 bis 9 sind unterschiedlich ausgeführte Knochenplatten 13 mit jeweils einer Durchgangsöffnung 14 für eine erfindungsgemäße Knochenschraube gezeigt. Fig. 4 zeigt den einfachsten Fall einer Knochenplatte mit einem axial durchgehenden Innengewinde 15.

Das Ausführungsbeispiel gemäß Fig. 5 weist zusätzlich zu dem Innengewinde 15 an ausschließlich einer Seite eine Senkung 16 für den Knochenschraubenkopf auf.

Das Ausführungsbeispiel gemäß Fig. 6 weist auf beiden Seiten jeweils eine Senkung 16 auf. In dem gezeigten Ausführungsbeispiel reduziert sich das Innengewinde 15 beispielhaft auf einem einzigen Gewindegang, wobei auch eine Ausführung mit mehreren Gewindegängen möglich ist.

Das Ausführungsbeispiel einer Knochenplatte 13 gemäß Fig. 7 weist kein Innengewinde auf, sondern zwei umfangsgeschlossene und axial beabstandete Flankenringe 17 (alternativ können auch mehr als zwei Flankenringe verankert bzw. vorgesehen werden).

Bei dem Ausführungsbeispiel gemäß Fig. 8 ist ebenfalls kein Innengewinde vorgesehen, sondern wiederum zwei axial benachbarte Flankenringe, wobei sich der Innendurchmesser der Flankenringe 17 unterscheidet (alternativ können auch mehr als zwei Flankenringe verankert bzw. vorgesehen werden).

Bei dem Ausführungsbeispiel gemäß Fig. 9 sind im Wesentlichen nur zwei Senkungen 16 vorgesehen, die einen Flankenring 17 begrenzen.

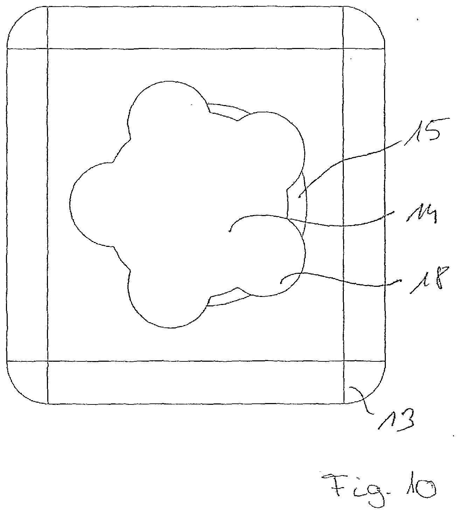

In Fig. 10 ist eine Knochenplatte 13 in einer Draufsicht gezeigt. Zu erkennen sind die Durchgangsöffnung 14 mit Innengewinde 15, wobei die Durchgangsöffnung 14 im Bereich ihres Außenumfangs mit mehreren gleichmäßig in Umfangsrichtung beabstandeten Einkerbungen 18 versehen ist, um die Stabilität der Verbindung aus Knochenschraube und Knochenplatte zu verbessern. Die Durchgangsöffnung 14 und die Einkerbungen 18 sind zylindrisch, d.h. verjüngen sich nicht. Die Einkerbungen besitzen einen teilkreisförmigen Querschnitt.

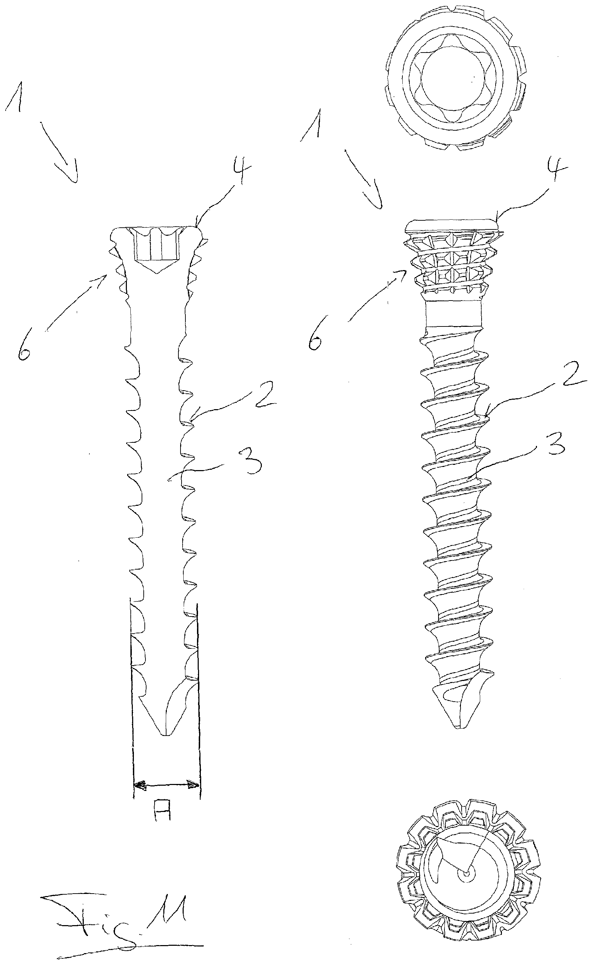

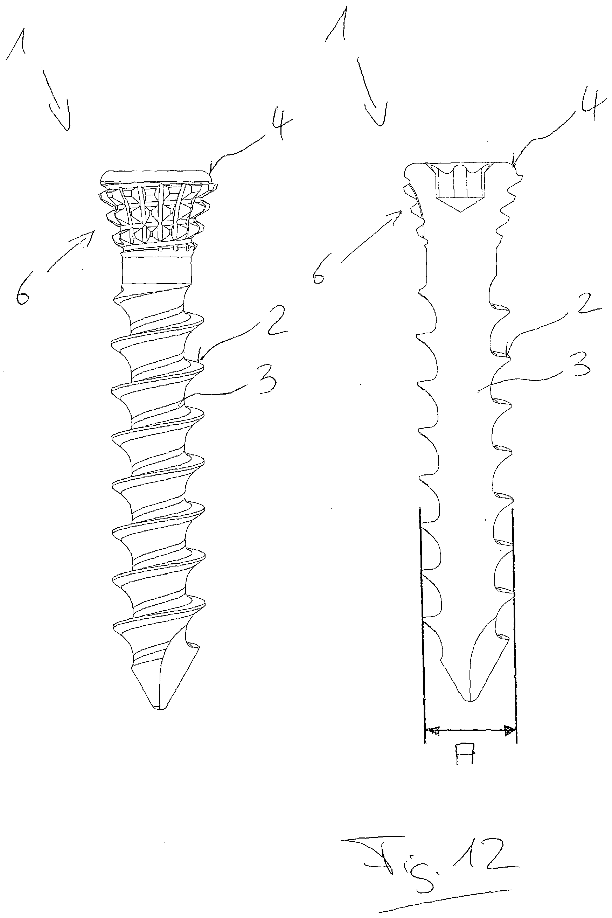

Die Fig. 11 und 12 zeigen jeweils eine vollständige erfindungsgemäße Knochenschraube 1, und zwar in einer Vorderansicht und in einem Schnitt entlang der Längsmittelachse, wobei der Außendurchmesser A des Schaftes 3 - also gemessen am Knochengewinde 2 - bei der Schraube 1 in Fig. 11 einen Wert von 2,7mm und bei der Schraube 1 in Fig. 12 einen Wert von 3,5mm besitzt. Die Rillenstruktur 6 am Schraubenkopf 4 ist hier jeweils in Form eines Gewindes vorgesehen, d.h. die Erhebungen der Rillenstruktur 6 bilden einen umlaufenden Gewindegang. Die Einkerbungen verlaufen jeweils parallel zur Längsmittelachse. In Fig. 11 ist außerdem - gegenüber der Vorderansicht und der Schnittansicht vergrößert - rechts oben eine Draufsicht auf den Schraubenkopf 2 und rechts unten eine Draufsicht auf die Spitze der Schraube 1 gezeigt.

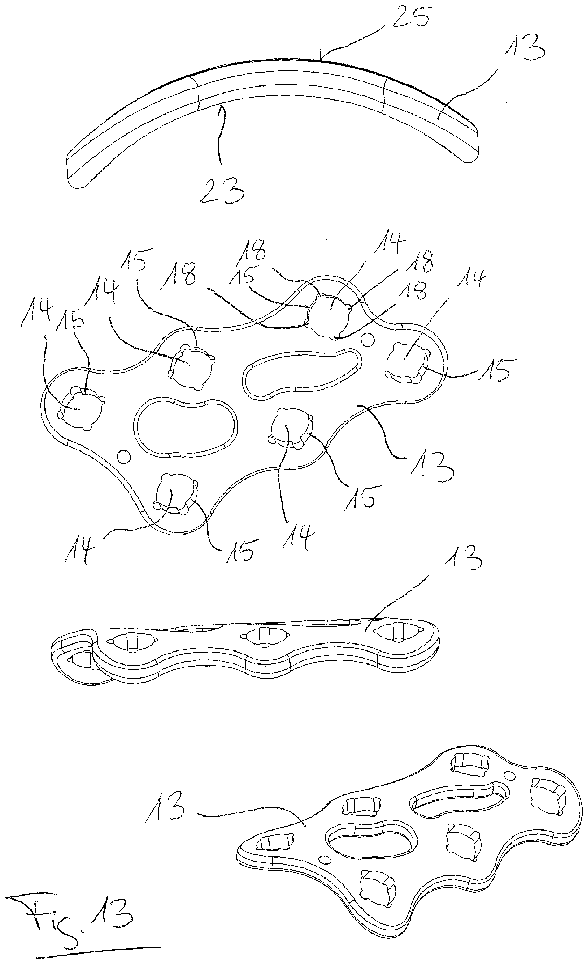

Fig. 13 zeigt ein Beispiel für eine Knochenplatte 13 mit hier sechs Durchgangsöffnungen 14, in die jeweils eine erfindungsgemäße Knochenschraube 1 eingedreht werden kann.

Die die Rillenstruktur 6 am Schraubenkopf 4 bildenden Erhebungen 9 wirken dabei mit einem Innengewinde 15 der jeweiligen Durchgangsöffnung 14 zusammen, das durch vier gleichmäßig in Umfangsrichtung voneinander beabstandete Einkerbungen 18 mit teilkreisförmigem Querschnitt unterbrochen ist. Mit den dadurch gebildeten Gewindeabschnitten 15 wirken die Erhebungen 9 des Schraubenkopfes 4 beim Eindrehen der Knochenschraube blockierend zusammen, indem die aus einem relativ härteren Material bestehenden Erhebungen 9 der Knochenschraube 1 die aus einem relativ weicheren Material bestehenden Gewindeabschnitte 15 der Durchgangsöffnung 14 verformen bzw. umformen.

Die Durchgangsöffnungen 14 sind zylindrisch, besitzen also jeweils - abgesehen von ihrem Innengewinde 15 und den Einkerbungen 18 - einen über ihre Längserstreckung durch die Knochenplatte 13 hindurch konstanten Innendurchmesser.

Wie die Seitenansicht in Fig. 13 zeigt, besitzt die Knochenplatte 13 eine konkave, im implantierten Zustand dem Knochen zugewandte Unterseite 23 und eine konvexe Oberseite 25.

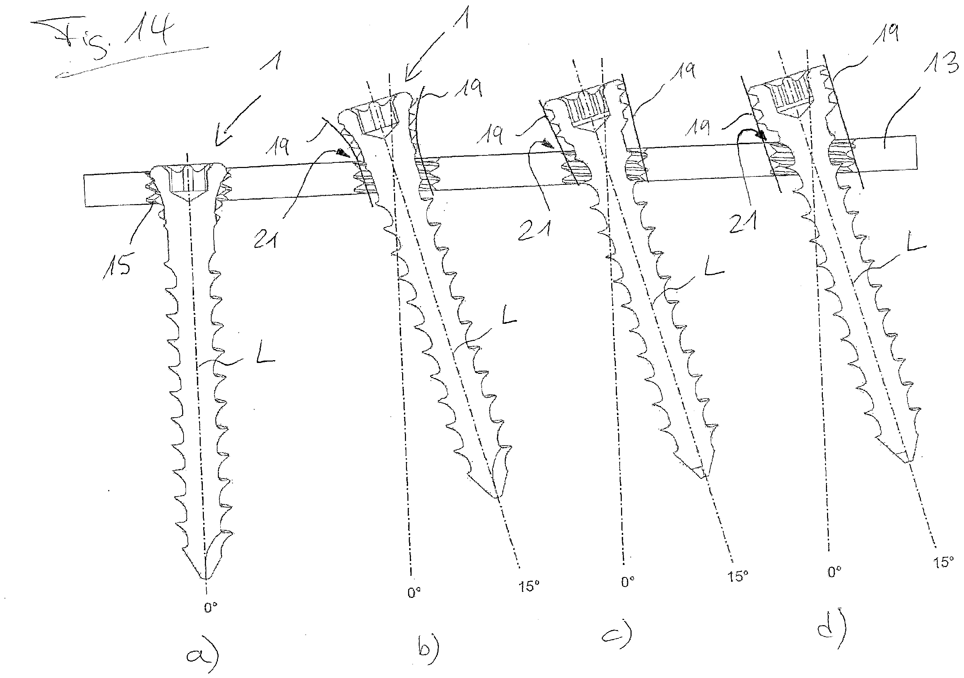

Fig. 14 veranschaulicht, dass eine erfindungsgemäße Knochenschraube 1 (Fig. 14a und 14b) mit konkaver Hüllkontur am Schraubenkopf problemlos bis zu einem Winkel eingedreht werden kann, der größer ist als bei nicht zur Erfindung gehörenden Schrauben mit konischer (Fig. 14c) oder zylindrischer (Fig. 14d) Hüllkontur am Schraubenkopf.

Der Vergleich der Fig. 14b, 14c und 14d zeigt, dass bei einem hier beispielhaft gewählten Eindrehwinkel von 15° der erfindungsgemäß konkave Schraubenkopf (Fig. 14b) problemlos eingedreht werden kann, da zwischen der durch die Konturlinien 19 veranschaulichten Hüllkontur des Schraubenkopfes und dem Innengewinde 15 der Durchgangsöffnung, insbesondere der Kante 21 am Eintrittsbereich der Durchgangsöffnung, genügend "Luft" vorhanden ist. Dies ist bei den nicht erfindungsgemäßen Schrauben nicht der Fall: Während eine Schraube mit konischer Kopfkontur (Fig. 14c) eventuell noch unter hohem Kraftaufwand eingedreht werden könnte, würde das Eindrehen einer Schraube mit zylindrischer Kopfkontur (Fig. 14d) zu einer Beschädigung der Schraub oder Platte oder - wie eingangs bereits erwähnt - die Gefahr einer Selbstzentrierung oder eines unkontrollierten Wegdrückens der Platte bedeuten.

Aus Fig. 14a geht hervor, dass die erfindungsgemäße Schraube 1 auch unter einem Winkel von 0° problemlos eingedreht werden kann.

### Bezugszeichenliste

- 1: Knochenschraube
- 2: Knochengewinde
- 3: Schaft
- 4: Schraubenkopf
- 5: Hüllkontur
- 6: Rillenstruktur
- 7: Flankenstrukturabschnitt(e)
- 8: Einkerbung(en)
- 9: Erhebungen
- 10: Flanke(n)
- 11: Flanke(n)
- 12: Kante
- 13: Knochenplatte
- 14: Durchgangsöffnung
- 15: Innengewinde
- 16: Senkung(en)
- 17: Flankenring(e)
- 18: Einkerbunge(n)
- 19: Konturlinie
- 21: Kante
- 23: Unterseite
- 25: Oberseite

- L: Längsmittelachse
- R: Radius
- A: Außendurchmesser

## Patentansprüche

1. Winkelstabiles Implantationssystem mit zumindest einer chirurgischen Knochenschraube (1) sowie mit einer Knochenplatte (13), die wenigstens eine Durchgangsöffnung (14) für die Knochenschraube (1) aufweist,
wobei die Knochenschraube (1) umfasst:
einen ein Knochengewinde (2) aufweisenden Schaft (3), Verblockungsmittel zum Zusammenwirken mit der Knochenplatte (13), sowie einen eine rotationssymmetrische Hüllkontur (5) aufweisenden Schraubenkopf (4);
wobei die Verblockungsmittel eine an dem Schraubenkopf (4) ausgebildete Rillenstruktur (6) mit mindestens einem sich in Umfangsrichtung erstreckenden Flankenstrukturabschnitt (7) aufweisen, und
wobei der sich in Umfangsrichtung erstreckende Flankenabschnitt der Rillenstruktur (6) in Umfangsrichtung mindestens einmal unterbrochen ist, derart dass mindestens eine Erhebung (9) zum blockierenden Zusammenwirken mit der Knochenplatte (13) resultiert,
**dadurch gekennzeichnet, dass** die Hüllkontur einen in axialer Richtung stetigen Verlauf aufweist, der von einer um eine Längsmittelachse (L) der Knochenschraube (1) rotierte, gekrümmte mathematische Funktion beschrieben ist.

2. System nach Anspruch 1,
**dadurch gekennzeichnet,**
**dass** das Material der Knochenschraube (1) härter ist als das Material der Knochenplatte (13).

3. System nach Anspruch 1 oder 2,
**dadurch gekennzeichnet,**
**dass** die Durchgangsöffnung (14) mit einem, insbesondere metrischen, Innengewinde (15) oder mit mindestens einem Flankenring (17) versehen ist, der insbesondere in einer Radialebene verläuft, und/oder dass die Durchgangsöffnung (14) einseitig oder beidseitig mit einer Senkung (16) versehen ist, und/oder dass die Durchgangsöffnung (14) zylindrisch ist.

4. System nach einem der Ansprüche 1 bis 3,
**dadurch gekennzeichnet,**
**dass** die Durchgangsöffnung (14) mit mindestens einer Längseinkerbung (8, 18) versehen ist, und/oder dass die Durchgangsöffnung (14) mit mehreren, insbesondere gleichmäßig in Umfangsrichtung voneinander beabstandeten, Längseinkerbungen (8, 18) versehen ist.

5. System nach Anspruch 4,
**dadurch gekennzeichnet,**
**dass** die mindestens eine Längseinkerbung (8, 18) in einer Ebene, die senkrecht zu einer Längsachse der Durchgangsöffnung (14) verläuft, einen teilkreisförmigen Querschnitt aufweist, und/oder dass die mindestens eine Einkerbung (8, 18) eine senkrecht zur Längserstreckung der Durchgangsöffnung (14) gemessene Tiefe besitzt, wobei die mindestens eine Einkerbung (8, 18) einen sich über ihre Axialerstreckung verändernden Tiefenverlauf aufweist und/oder dass mindestens zwei Einkerbungen (8, 18) unterschiedlich tief sind, und/oder dass die mindestens eine Einkerbung (8, 18) zylindrisch ist.

6. System nach einem der vorhergehenden Ansprüche,
**dadurch gekennzeichnet,**
**dass** die Rillenstruktur (6) als Außengewinde und sich der gewendelte Flankenabschnitt über mindestens den 1-fachen Umfang des Schraubenkopfes (4) erstreckt, oder dass mindestens ein ringförmiger Flankenstrukturabschnitt (7) vorgesehen ist, wobei insbesondere mehrere zueinander parallele ringförmige Flankenstrukturabschnitte (7) vorgesehen sind, die sich entweder in zueinander parallelen Radialebenen befinden oder die winklig zu einer Radialebene angeordnet sind.

7. System nach einem der vorhergehenden Ansprüche,
**dadurch gekennzeichnet,**
**dass** die Hüllkontur (5) des Schraubenkopfes (4) zumindest abschnittsweise trompetenförmig konturiert ist, wobei insbesondere die Hüllkontur (5) des Schraubenkopfes (4) über den größten Teil der Axialerstreckung des Schraubenkopfes (4), insbesondere über die gesamte Axialerstreckung des Schraubenkopfes (4) konkav ausgebildet ist.

8. System nach einem der vorhergehenden Ansprüche,
**dadurch gekennzeichnet,**
**dass** die Mantelfläche der Hüllkontur (5) des Schraubenkopfes (4) in einer Längsschnittansicht einen Teilkreisbogen umfasst, wobei insbesondere der Teilkreisbogen einen Radius (R) aus einem Wertebereich zwischen 0,5 und 7mm, insbesondere zwischen 1 und 6mm, insbesondere zwischen 2 und 4mm, insbesondere von 3mm, besitzt.

9. System nach einem der Ansprüche 1 bis 7,
**dadurch gekennzeichnet,**
**dass** die Hüllkontur (5) des Schraubenkopfes (4) mindestens zwei Kegelstumpfabschnitte mit voneinander unterschiedlichen Kegelwinkeln umfasst.

10. System nach einem der vorhergehenden Ansprüche,
**dadurch gekennzeichnet,**
**dass** mehrere axial beabstandete Erhebungen (9) vorgesehen sind.

11. System nach einem der vorhergehenden Ansprüche,
**dadurch gekennzeichnet,**
**dass** der mindestens eine Flankenstrukturabschnitt (7) durch mehrere Einkerbungen (8, 18) unterbrochen ist, die in einer Projektion sich axial erstrecken oder winklig zur Axialerstreckung angeordnet sind, wobei insbesondere die Einkerbungen (8, 18) gleichmäßig in Umfangsrichtung verteilt angeordnet sind, wobei insbesondere die Einkerbungen (8, 18) sich bis zu einem freien Ende des Schraubenkopfes (4) erstrecken.

12. System nach einem der vorhergehenden Ansprüche,
**dadurch gekennzeichnet,**
**dass** die mindestens eine Erhebung (9) mindestens zwei, insbesondere genau vier zu einer Erhebungsmitte hin geneigte, Flanken (10, 11) aufweist, und/oder dass die mit ihren Flächenseiten in Umfangsrichtung weisenden Flanken (10, 11) der mindestens einen Erhebung (9) bezüglich einer Längsmittelachse (L) der Knochenschraube sich gerade in radialer Richtung erstrecken oder zur Radialrichtung geneigt sind, insbesondere derart, dass die mindestens eine Erhebung in einer Querschnittsansicht der Knochenschraube trapezförmig konturiert ist.

13. System nach einem der vorhergehenden Ansprüche,
**dadurch gekennzeichnet,**
**dass** die mindestens eine Erhebung (9) eine Pyramidenform, eine Walmdachform, eine Pyramidenstumpfform, eine Keilform oder eine Keilstumpfform aufweist, und/oder dass eine am freien Ende des Schraubenkopfes (4) vorgesehene Umfangskante gerundet ist, und/oder dass die Mantelfläche der Hüllkontur (5) einen stetigen konkaven Verlauf aufweist.

14. System nach einem der vorhergehenden Ansprüche,
**dadurch gekennzeichnet,**
**dass** der sich in Umfangsrichtung erstreckende Flankenabschnitt der Rillenstruktur (6) in Umfangsrichtung mehrfach unterbrochen ist, derart dass eine Mehrzahl von in Umfangrichtung beabstandeten Erhebungen (9) zum blockierenden Zusammenwirken mit der Knochenplatte (13) oder dem Knochen resultieren.

## Claims

1. An angle stable implant system having at least one surgical bone screw (1) as well as having a bone plate (13) which has at least one passage opening (14) for the bone screw (1),
wherein the bone screw (1) comprises:
a shaft (3) having a bone thread (2); blocking means for cooperating with the bone plate (13); and a screw head (4) having a rotationally symmetrical enveloping contour (5),
wherein the blocking means have a groove structure (6) which is formed at the screw head (4) and which has at least one flank structure section (7) extending in the peripheral direction, and
wherein the flank section of the groove structure (6) extending in the peripheral direction is interrupted at least once in the peripheral direction such that at least one elevated portion (9) results for blocking cooperation with the bone plate (13),
**characterized in that**
the enveloping contour has a constant progression in the axial direction that is described by a curved mathematical function rotated about a central longitudinal axis (L) of the bone screw (1).

2. A system in accordance with claim 1,
**characterized in that**
the material of the bone screw (1) is harder than the material of the bone plate (13).

3. A system in accordance with claim 1 or claim 2,
**characterized in that**
the passage opening (14) is provided with an internal thread (15), in particular with a metric internal thread, or is provided with at least one flank ring (17) which in particular extends in a radial plane; and/or **in that** the passage opening (14) is provided with a depression (16) at one side or at both sides; and/or **in that** the passage opening (14) is cylindrical.

4. A system in accordance with any one of the claims 1 to 3,
**characterized in that**
the passage opening (14) is provided with at least one longitudinal notch (8, 18); and/or **in that** the passage opening (14) is provided with a plurality of longitudinal notches (8, 18) which are in particular evenly spaced apart from one another in the peripheral direction.

5. A system in accordance with claim 4,
**characterized in that**
the at least one longitudinal notch (8, 18) has a part circular cross-section in a plane which extends perpendicular to a longitudinal axis of the passage opening (14); and/or **in that** the at least one notch (8, 18) has a depth measured perpendicular to the longitudinal extent of the passage opening (14), with the at least one notch (8, 18) having a depth progression varying over its axial extent; and/or in that **in that** at least two notches (8, 18) are of different depths; and/or **in that** the at least one notch (8, 18) is cylindrical.

6. A system in accordance with any one of the preceding claims,
**characterized in that**
the groove structure (6) extends as an external thread and the coiled flank section extends over at least once the periphery of the screw head (4); or **in that** at least one ring-shaped flank structure section (7) is provided, with in particular a plurality of mutually parallel, ring-shaped flank structure sections (7) being provided which are either located in mutually parallel radial planes or which are arranged at an angle to a radial plane.

7. A system in accordance with any one of the preceding claims,
**characterized in that**
the enveloping contour (5) of the screw head (4) is contoured at least sectionally in the form of a trumpet, with in particular the enveloping contour (5) of the screw head (4) being formed as concave over the larger part of the axial extent of the screw head (4), in particular over the total axial extent of the screw head (4).

8. A system in accordance with any one of the preceding claims,
**characterized in that**
the jacket surface of the enveloping contour (5) of the screw head (4) comprises a part arc of a circle in a longitudinal sectional view, with in particular the part arc of a circle having a radius (R) from a value range between 0.5 and 7 mm, in particular between 1 and 6 mm, in particular between 2 and 4 mm, in particular of 3 mm.

9. A system in accordance with any one of the claims 1 to 7,
**characterized in that**
the enveloping contour (5) of the screw head (4) comprises at least two frustoconical sections having mutually different conical angles.

10. A system in accordance with any one of the preceding claims,
**characterized in that**
a plurality of axially spaced apart elevated portions (9) are provided.

11. A system in accordance with any one of the preceding claims,
**characterized in that**
the at least one flank structure section (7) is interrupted by a plurality of notches (8, 18) which extend axially in a projection or which are arranged at an angle to the axial extent, with in particular the notches (8, 18) being arranged evenly distributed in the peripheral direction, with in particular the notches (8, 18) extending up to a free end of the screw head (4).

12. A system in accordance with any one of the preceding claims,
**characterized in that**
the at least one elevated portion (9) has at least two flanks (10, 11), in particular exactly four flanks, inclined with respect to an elevated portion center; and/or **in that** the surface sides of the flanks (10, 11) of the at least one elevated portion (9) facing in the peripheral direction extend in a straight line in a radial direction with respect to a central longitudinal axis (L) of the bone screw or are inclined with respect to the radial direction, in particular such that the at least one elevated portion is contoured in trapezoid shape in a cross-sectional view of the bone screw.

13. A system in accordance with any one of the preceding claims,
**characterized in that**
the at least one elevated portion (9) has a pyramid shape, a hipped roof shape, a shape of a truncated pyramid, a wedge shape or a frustoconical shape; and/or **in that** a peripheral edge provided at the free end of the screw head (4) is rounded; and/or **in that** the jacket surface of the enveloping contour (5) has a constant concave progression.

14. A system in accordance with any one of the preceding claims,
**characterized in that**
the flank section of the groove structure (6) extending in the peripheral direction is interrupted a multiple of times in the peripheral direction such that a plurality of elevated portions (9) spaced apart in the peripheral direction result for the blocking cooperation with the bone plate (13) or with the bone.

## Revendications

1. Système d'implantation angulairement stable, comportant au moins une vis chirurgicale à os (1) ainsi qu'une plaque à os (13) qui présente au moins une ouverture traversante (14) pour la vis à os (1),
la vis à os (1) comprenant :
une tige (3) pourvue d'un pas de vis à os (2), des moyens de blocage pour la coopération avec la plaque à os (13) ainsi qu'une tête de vis (4) présentant un contour enveloppe (5) à symétrie de révolution ;
dans lequel
les moyens de blocage présentent une structure rainurée (6) réalisée sur la tête de vis (4) et pourvue d'au moins une portion de structure à flancs (7) qui s'étend en direction périphérique, et
la portion à flancs de la structure rainurée (6) s'étendant en direction périphérique est interrompue au moins une fois en direction périphérique, de telle sorte qu'il en résulte au moins un bossage (9) pour la coopération par blocage avec la plaque à os (13),
**caractérisé en ce que**
le contour enveloppe présente une allure constante en direction axiale qui est décrite par une fonction mathématique incurvée tournée autour d'un axe médian longitudinal (L) de la vis à os (1).

2. Système selon la revendication 1,
**caractérisé en ce que**
le matériau de la vis à os (1) est plus dur que le matériau de la plaque à os (13).

3. Système selon la revendication 1 ou 2,
**caractérisé en ce que**
l'ouverture traversante (14) est pourvue d'un taraudage (15) en particulier métrique ou d'au moins un anneau de flanc (17) qui s'étend en particulier dans un plan radial,
et/ou **en ce que** l'ouverture traversante (14) est pourvue d'un lamage (16) sur un côté ou sur les deux côtés,
et/ou **en ce que** l'ouverture traversante (14) est cylindrique.

4. Système selon l'une des revendications 1 à 3,
**caractérisé en ce que**
l'ouverture traversante (14) est pourvue d'au moins une entaille longitudinale (8, 18),
et/ou **en ce que** l'ouverture traversante (14) est pourvue de plusieurs entailles longitudinales (8, 18) espacées les unes des autres en particulier régulièrement en direction périphérique.

5. Système selon la revendication 4,
**caractérisé en ce que**
dans un plan qui s'étend perpendiculairement à un axe longitudinal de l'ouverture traversante (14), ladite au moins une entaille longitudinale (8, 18) présente une section transversale en forme de cercle partiel, et/ou
**en ce que** ladite au moins une entaille (8, 18) possède une profondeur mesurée perpendiculairement à l'extension longitudinale de l'ouverture traversante (14), ladite au moins une entaille (8, 18) présente une allure en profondeur qui se modifie sur son extension axiale, et/ou en ce qu'au moins deux entailles (8, 18) présentent des profondeurs différentes, et/ou
**en ce que** ladite au moins une entaille (8, 18) est cylindrique.

6. Système selon l'une des revendications précédentes,
**caractérisé en ce que**
la structure rainurée (6) s'étend sous forme de filetage et la portion à flancs héliocoïdale s'étend au moins sur 1 fois la périphérie de la tête de vis (4), ou
**en ce qu'**il est prévu au moins une portion de structure à flancs (7) annulaire, et en particulier il est prévu plusieurs portions de structure à flancs (7) annulaires parallèles les unes aux autres qui se trouvent soit dans des plans radiaux parallèles les uns aux autres soit sous un angle par rapport à un plan radial.

7. Système selon l'une des revendications précédentes,
**caractérisé en ce que**
le contour enveloppe (5) de la tête de vis (4) est profilé au moins localement en forme de trompette, et en particulier le contour enveloppe (5) de la tête de vis (4) est réalisé concave sur la plus grande partie de l'extension axiale de la tête de vis (4), en particulier sur la totalité de l'extension axiale de la tête de vis (4).

8. Système selon l'une des revendications précédentes,
**caractérisé en ce que**
la surface enveloppe du contour enveloppe (5) de la tête de vis (4) présente en vue en coupe longitudinale un arc de cercle partiel, et en particulier l'arc de cercle partiel présente un rayon (R) d'une plage de valeurs comprise entre 0,5 et 7 mm, en particulier entre 1 et 6 mm, en particulier entre 2 et 4 mm, en particulier de 3 mm.

9. Système selon l'une des revendications 1 à 7,
**caractérisé en ce que**
le contour enveloppe (5) de la tête de vis (4) comprend au moins deux portions tronconiques ayant des angles de cône qui diffèrent l'un de l'autre.

10. Système selon l'une des revendications précédentes,
**caractérisé en ce que**
il est prévu plusieurs bossages (9) axialement espacés.

11. Système selon l'une des revendications précédentes,
**caractérisé en ce que**
ladite au moins une portion de structure à flancs (7) est interrompue par plusieurs entailles (8, 18) qui s'étendent, dans une projection, axialement ou sous un angle par rapport à l'extension axiale, et en particulier les entailles (8, 18) sont disposées en étant réparties régulièrement en direction périphérique, et en particulier les entailles (8, 18) s'étendent jusqu'à une extrémité libre de la tête de vis (4).

12. Système selon l'une des revendications précédentes,
**caractérisé en ce que**
ledit au moins un bossage (9) comprend au moins deux, en particulier précisément quatre flancs (10, 11) inclinés vers un centre de bossage, et/ou
**en ce que** les flancs (10, 11) ayant leurs faces surfaciques dirigées en direction périphérique dudit au moins un bossage (9) s'étendent en ligne droite en direction radiale par rapport à un axe médian longitudinal (L) de la vis à os ou sont inclinés par rapport à la direction radiale, en particulier de telle sorte que ledit au moins un bossage est profilé en forme trapézoïdale, en vue en coupe transversale de la vis à os.

13. Système selon l'une des revendications précédentes,
**caractérisé en ce que**
ledit au moins un bossage (9) présente une forme pyramidale, une forme de toiture en croupe, une forme de pyramide tronquée, une forme de coin ou une forme de coin tronqué, et/ou en ce qu'une arête périphérique prévue à l'extrémité libre de la tête de vis (4) est arrondie, et/ou
**en ce que** la surface enveloppe du contour enveloppe (5) présente une allure concave constante.

14. Système selon l'une des revendications précédentes,
**caractérisé en ce que**
la portion de flanc de la structure rainurée (6) qui s'étend en direction périphérique est interrompue plusieurs fois en direction périphérique, de telle sorte qu'il en résulte une pluralité de bossages (9) espacés en direction périphérique pour la coopération de blocage avec la plaque à os (13) ou avec l'os.
